(19)

Europäisches Patentamt

European Patent Office

Office européen des brevets

(11) **EP 2 689 727 A1**

(12) **EUROPEAN PATENT APPLICATION**
published in accordance with Art. 153(4) EPC

(43) Date of publication:
**29.01.2014 Bulletin 2014/05**

(51) Int Cl.:
***A61B 6/00*** *(2006.01)*       ***G06T 5/10*** *(2006.01)*

(21) Application number: **12761309.9**

(22) Date of filing: **23.03.2012**

(86) International application number:
**PCT/JP2012/002012**

(87) International publication number:
**WO 2012/127874 (27.09.2012 Gazette 2012/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.03.2011 JP 2011065510**

(71) Applicant: **FUJIFILM Corporation Tokyo 106-8620 (JP)**

(72) Inventor: **IMAI, Yoshiro Ashigarakami-gun Kanagawa 258-8538 (JP)**

(74) Representative: **Klunker . Schmitt-Nilson . Hirsch Patentanwälte Destouchesstrasse 68 80796 München (DE)**

(54) **IMAGE PROCESSING DEVICE, IMAGE PROCESSING METHOD, AND IMAGE PROCESSING PROGRAM**

(57)     [Aim] To appropriately determine passband characteristics for suppressing a periodic pattern, such as a grid image, in a radiographic image including the periodic pattern.

[Solution Means] A peak position (pf) and a peak width (pw) of a frequency spectrum corresponding to a periodic pattern are detected by performing, with respect to at least one direction, frequency analysis on an image signal representing an image including the periodic pattern. The passband characteristics (F) of a one-dimensional filter suppressing a spatial frequency component corresponding to the periodic pattern in the image signal are determined based on the detected peak position (pf) and the detected peak width (pw).

## FIG.9A

**Description**

BACKGROUND OF THE INVENTION

Field of the Invention

The present invention relates to an image processing apparatus, method and program that suppresses a spatial frequency component corresponding a periodic pattern in an image signal. In particular, the present invention relates to an image processing apparatus, method and program that suppresses a periodic pattern in a radiographic image caused by a grid used in radiography.

Description of the Related Art

Conventionally, storable phosphors (photostimulable phosphors) have been used. When a storable phosphor is irradiated with radiation (X-rays, α-rays, β-rays, γ-rays, an electron beam, ultraviolet rays or the like), a part of radiation energy is stored in the storable phosphor. After then, when excitation light, such as visible light and a laser beam, is output to the storable phosphor, the storable phosphor emits photoluminescence corresponding to the radiation energy stored therein. For example, a radiographic image readout apparatus using the storable phosphor is widely used in CR (Computed Radiography). A storable phosphor sheet, in which the storable phosphor is deposited on a substrate, is irradiated with radiation that has passed through a subject, such as a human body, and radiographic information is temporarily stored and recorded on the storable phosphor sheet. Excitation light, such as a laser beam, is output to the storable phosphor sheet to induce photoluminescence. Further, photoelectric conversion is performed on the photoluminescence to obtain an image signal.

Here, when a radiographic image of a subject is imaged and recorded on the storable phosphor sheet or the like, imaging is performed by placing a grid between the subject and the sheet in some cases so that radiation scattered by the subject does not irradiate the sheet. For example, lead or the like, which does not pass radiation therethrough, and aluminum, wood or the like, which tends to pass radiation therethrough, are alternately arranged at a narrow pitch of about 4 line/mm in the grid. When radiography is performed by using the grid, radiation scattered by the subj ect does not tend to irradiate the sheet. Therefore, it is possible to improve the contrast of the radiographic image of the subject. However, since a grid image is included in the image, there is a problem that the image quality deteriorates. Further, when the size of the image including the grid image is enlarged or reduced, aliasing due to folding occurs depending on the magnification or reduction ratio. Further, when aliasing overlaps with the spatial frequency of the grid image or the like, a narrow stripe pattern (moire) is generated, and observation of a regenerated image becomes difficult.

Patent Document 1 discloses a method for removing a grid image, as a method for removing such a periodic pattern from an image. In the method, one-dimensional high-pass filter processing is performed on the image in a direction in which the stripe pattern of the grid image is arranged. Further, one-dimensional low-pass filter processing is performed on the image in a direction parallel to the stripe pattern of the grid image. Accordingly, a spatial frequency component corresponding to the grid image is extracted from an original image, and the extracted spatial frequency component is subtracted from the original image.

Further, Patent Document 2 discloses a method for obtaining an image in which an artifact caused by a grid is suppressed. In the method, frequency analysis is performed on image data by two-dimensional or one-dimensional Fourier transformation, and a peak position (frequency) of a moire stripe, a peak height, a full width at half maximum (HWFM), total energy at peak, the direction of the grid, and the like are obtained. Further, smoothing filtering is performed at a kernel size determined based on the pixel size of the image, the peak position and the energy at peak.

Further, Patent Document 3 discloses an image processing method. In the method, two-dimensional Fourier transformation is performed on image data to detect a spatial frequency component corresponding to a periodic pattern. Further, filtering is performed by using a filter that removes only the detected spatial frequency component.

Related Technical Documents

Patent Documents

Patent Document 1:

Japanese Unexamined Patent Publication No. 2003-150954

Patent Document 2:

U.S. Patent No. 6,269,176

Patent Document 3:

Japanese Unexamined Patent Publication No. 2009-195512

SUMMARY OF THE INVENTION

Here, there is a demand for removing a periodic pattern, such as moire, without removing components representing a subject as possible to obtain a high quality image appropriate for diagnosis based on the image. However, in the method disclosed in Patent Document 1, a spatial frequency component corresponding to a periodic pattern, such as a stripe pattern of a grid image, is detected by performing one-dimensional high-pass filtering processing. Therefore, if the periodic pattern is present not in a high frequency band but also in a low frequency band, it is impossible to detect the periodic pattern as intended. Therefore, it has been impossible to adopt the method disclosed in Patent Document 1.

Further, in the method disclosed in Patent Document 2, a localized noise component may be suppressed by performing smoothing filtering processing. However, the method removes not only a detected spatial frequency component corresponding to the periodic pattern from the image. Therefore, it has been impossible to intensively remove the spatial frequency component including the periodic pattern.

In the method disclosed in Patent Document 3, the load of calculation is high, because a spatial frequency component corresponding a periodic pattern is detected by two-dimensional Fourier transformation. Therefore, there has been a demand for a method for determining the passband characteristics of the spatial frequency component corresponding to the periodic pattern with a lower calculation load.

In view of the foregoing circumstances, it is an object of the present invention to provide an image processing apparatus, an image processing method and an image processing program that can appropriately determine passband characteristics with a low calculation load even if a frequency component corresponding to a periodic pattern is present in a middle frequency band or a low frequency band.

An image processing apparatus of the present invention is an image processing apparatus comprising:

a periodic pattern detection means that detects a peak position and a peak width of a frequency spectrum corresponding to a periodic pattern by performing, with respect to at least one direction, frequency analysis on an image signal representing an image including the periodic pattern; and a passband characteristics determination means that determines, based on the detected peak position and the detected peak width, the passband characteristics of a one-dimensional filter suppressing a spatial frequency component corresponding to the periodic pattern in the image signal.

An image processing method of the present invention is an image processing method comprising the steps of:

detecting a peak position and a peak width of a frequency spectrum corresponding to a periodic pattern by performing, with respect to at least one direction, frequency analysis on an image signal representing an image including the periodic pattern; and determining, based on the detected peak position and the detected peak width, the passband characteristics of a one-dimensional filter suppressing a spatial frequency component corresponding to the periodic pattern in the image signal.

An image processing program of the present invention is an image processing program for causing a computer to function as:

a periodic pattern detection means that detects a peak position and a peak width of a frequency spectrum corresponding to a periodic pattern by performing, with respect to at least one direction, frequency analysis on an image signal representing an image including the periodic pattern; and a passband characteristics determination means that determines, based on the detected peak position and the detected peak width, the passband characteristics of a one-dimensional filter suppressing a spatial frequency component corresponding to the periodic pattern in the image signal.

The term "periodic pattern" means a noise having a periodic pattern included in an original image. For example, the periodic pattern means a grid image, moire or the like included in an original image when a radiographic image is imaged on a storable phosphor sheet by using the grid.

Further, the term "passband characteristics" means the characteristics of a filter that passes or removes a predetermined band. The passband characteristics include both of band-pass characteristics (band pass characteristics) and band removal characteristics (band stop characteristics).

Further, an arbitrary peak position may be defined as long as the peak position represents a representative value of the peak frequency of a frequency spectrum. Similarly, an arbitrary peak width may be defined as long as the peak width represents a representative value of the peak width of a frequency spectrum.

The passband characteristics determination means may determine any kind of passband characteristics as long as the passband characteristics include a peak position of a frequency component corresponding a periodic pattern, and the peak position is included in a band to be passed, and the passband characteristics are determinable based on the peak position and the peak width.

It is desirable that the band-pass width is wider than or equal to a peak width to remove a frequency component corresponding to a periodic pattern. For example, it is desirable that the passband characteristics determination means determines the passband characteristics in such a manner that the peak position is included and that a passband width is wider as the peak width is wider.

In the passband characteristics, a low frequency band includes many subject components. Therefore, a risk of removing the subject components together with a frequency component corresponding to a periodic pattern is higher as the band-pass of the filter is wider in the low frequency band. Therefore, in the low frequency band, it is desirable that the passband characteristics include the peak of the periodic pattern and that the width of the passband is narrow. For example, the passband characteristics determination means may determine the passband characteristics in such a manner that a passband width is narrower as the peak position is located in a lower frequency band.

Further, the passband characteristics determination means may determine the passband characteristics by a normal distribution function defined by the peak position and the peak width. For example, the normal distribution function may be defined by using the peak position as the mean, and the peak width at half maximum, as standard deviation. A normal distribution curve that is enlarged or reduced in such a manner that the peak of the normal distribution function corresponds to 1 in frequency response may be determined as the passband characteristics.

The passband characteristics determination means may determine the passband characteristics by a rectangular function having a width wider than or equal to the peak width, and the center of the rectangular function being the peak position.

Further, the passband characteristics determination means may determine the passband characteristics based on the height of the peak. That is because when the magnitude of a spectrum is small, even if not an entire frequency band including a frequency component corresponding to a periodic pattern is suppressed (a part of the frequency component corresponding to the periodic pattern remains in the image signal), it is possible to suppress the periodic pattern in such a manner that the quality is sufficient to be used as an image for diagnosis. For example, the passband characteristics may be determined in such a manner that the band-pass frequency response of a band-pass filter is higher as the peak height is higher, and that the band-pass frequency response of the band-pass filter is lower as the peak height is lower.

It is desirable that an image processing apparatus according to the present invention further includes a suppression means that suppresses the periodic pattern in the image signal by performing one-dimensional filtering based on the determined passband characteristics.

Further, the passband characteristics determination means in an image processing apparatus according to the present invention may set an upper limit or a lower limit of a passband width in the passband characteristics. That is because filtering by a band-pass (or band-stop) filter that passes (or stops) only an extremely narrow band causes generation of an artifact or the like in an image, and that is not desirable. Further, filtering by a band-pass (band-stop) filter that passes a too wide band is not desirable because there is a risk of removing information, such as a subject component, which is important in diagnosis based on the image, together with a component corresponding to a periodic pattern.

The passband characteristics determination means may determine the passband characteristics in such a manner that the passband characteristics differ depending on an imaging condition of the image. Here, an imaged region of an image and an imaging condition of the image may be obtained by using an imaging menu, such as information about the imaging condition and the imaged region, which is specified at an imaging apparatus of an original image. Alternatively, imaging region information, such as an organ of a subject and a lesion, which has been input by a user through his/her manual operation may be used. Further, an image processing apparatus of the present invention may further include a region extraction means that extracts an imaged region of the image from the image, and use the extracted imaged region information. For example, methods disclosed in Japanese Unexamined Patent Publication No. 2002-109548 and Japanese Unexamined Patent Publication No. 2003-006661, which are proposed by the applicant of the present application, may be used. In the methods, a thorax is automatically detected by performing template matching using a template that is substantially similar to the outline of an average cardiothorax, as reference.

According to the image processing apparatus, the image processing method and the image processing program of the present invention, a peak position and a peak width of a frequency spectrum corresponding to a periodic pattern are detected by performing, with respect to at least one direction, frequency analysis on an image signal representing an image including the periodic pattern. Further, passband characteristics of a one-dimensional filter that suppresses a spatial frequency component corresponding to the periodic pattern in the image signal are determined based on the detected peak position and the detected peak width. Therefore, it is possible to appropriately determine, based on the peak position and the peak width, the passband characteristics of a one-dimensional filter that can appropriately suppress a frequency component without excessively suppressing a subject component in the image. Consequently, it is possible to obtain a high quality image with a relatively low calculation load.

BRIEF DESCRIPTION OF THE DRAWINGS

Figure 1 is a schematic diagram illustrating a radiography apparatus;
Figure 2 is a diagram illustrating a radiographic image obtained by imaging with a grid;

Figure 3 is a perspective view illustrating an example of a radiographic image readout apparatus;

Figure 4 is a diagram illustrating a relationship between scan directions and an image to be read out;

Figure 5 is a schematic diagram illustrating the configuration of an image processing apparatus according to an embodiment of the present invention;

Figure 6 is a flow chart illustrating processing by an image processing apparatus according to an embodiment of the present invention;

Figure 7 is a schematic diagram for explaining region extraction processing in an embodiment of the present invention;

Figure 8 is a diagram for explaining normalization processing in an embodiment of the present invention;

Figure 9A is a diagram for explaining passband characteristics determination processing in an embodiment of the present invention;

Figure 9B is a diagram for explaining a modified example of passband characteristics determination processing in an embodiment of the present invention;

Figure 10A is a diagram for explaining suppression processing in an embodiment of the present invention;

Figure 10B is a diagram for explaining a modified example of suppression processing in an embodiment of the present invention;

Figure 11 is a diagram illustrating a modified example of passband characteristics in an embodiment of the present invention (No. 1);

Figure 12 is a diagram illustrating a modified example of passband characteristics in an embodiment of the present invention (No. 2); and

Figure 13 is a diagram illustrating a modified example of passband characteristics in an embodiment of the present invention (No. 3).

DESCRIPTION OF THE PREFERRED EMBODIMENTS

Hereinafter, embodiments of the present invention will be described with reference to drawings. In the following embodiments, a case in which a periodic pattern suppression processing apparatus according to the present invention is used in a radiographic image readout apparatus will be described. Alternatively, the periodic pattern suppression processing apparatus may be used in an image processing apparatus or the like for suppressing a periodic pattern included in a photographic image that was obtained in ordinary photography using a digital camera or the like when photography was performed through a window screen, a blind or the like. Here, the radiographic image readout apparatus reads out, as a digital image signal, a radiographic image of a human body recorded on a storable phosphor sheet by scanning the radiographic image with a laser beam.

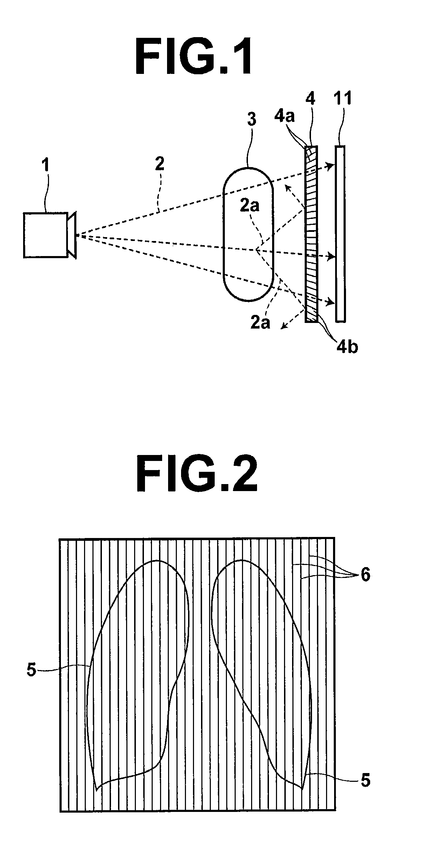

Figure 1 is a schematic diagram illustrating a radiography apparatus. Radiation 2 is output from a radiation source 1, and passes through a subject 3, and reaches a static grid (hereinafter, simply referred to as "grid") 4. In the grid 4, lead 4a, which absorbs the radiation 2, and aluminum 4b, which passes the radiation 2, are alternately arranged, for example, at a pitch of about 4 line/mm. Further, the lead 4a is set in such a manner that the inclination of the lead 4a is slightly different depending on its position so that the radiation 2 passes through the aluminum 4b, and enters a storable phosphor sheet 11.

Therefore, the radiation 2 that has passed through the subject 3 is absorbed by the lead 4a, and does not reach the storable phosphor sheet 11. However, the radiation 2 passes through the aluminum 4b, and reaches the storable phosphor sheet 11. A grid image of a stripe pattern of 4 line/mm is recorded on the storable phosphor sheet 11 together with a subject image. Meanwhile, scattered radiation 2a, which is scattered in the subject 3, is absorbed by the lead 4a, which is set in such a manner to be inclined depending on its position, or reflected by the surface of the grid 4. Therefore, the scattered radiation 2a does not reach the storable phosphor sheet 11. Hence, the storable phosphor sheet 11 can record a sharp radiographic image with a little amount of scattered radiation 2a irradiating the storable phosphor sheet 11. Figure 2 is a diagram illustrating an example of a radiographic image of a subject image 5 and a moire pattern 6 caused by a grid image. The radiographic image is stored and recorded on the storable phosphor sheet 11 when radiography is performed by using the radiography apparatus illustrated in Figure 1.

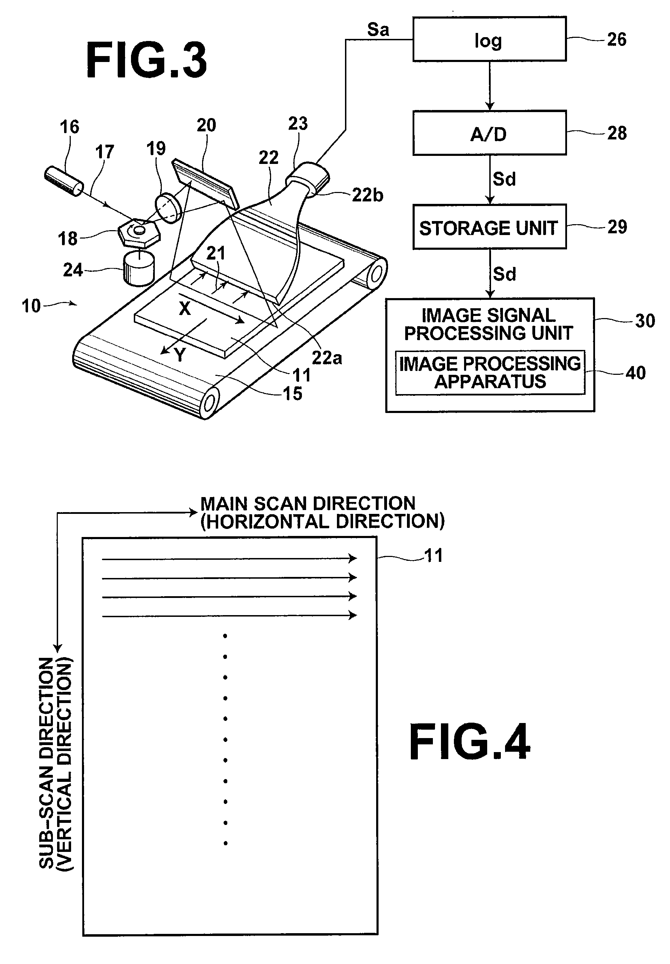

Figure 3 is a diagram illustrating a perspective view and a functional block diagram of a radiographic image readout apparatus in combination. The storable phosphor sheet 11 is set at a predetermined position of a readout unit 10, and conveyed by a sheet conveyance means 15, such as an endless belt, which is driven by a drive means (not illustrated). The storable phosphor sheet 11 is conveyed (sub-scanned) in the direction of arrow Y, for example, at a scan pitch of 10 line/mm. Meanwhile, a light beam 17 is output from a laser beam source 16, and reflected by a rotary polyhedral mirror 18 toward a condensing lens 19, such as an fθ lens. The rotary polyhedral mirror 18 is driven by a motor 24, and rotates at high speed in the direction of an arrow. After the light beam 17 passes through the condensing lens 19, the light beam 17 is reflected by a mirror 20 toward the storable phosphor sheet 11. The storable phosphor sheet 11 is main-scanned by the light beam 17 in the direction of arrow X, which is at a substantially right angle to the sub-scan direction (the direction of arrow Y).

When the storable phosphor sheet 11 is illuminated with the light beam 17, stimulated emission light 21 in an amount corresponding to radiographic image information stored and recorded at an illuminated position of the storable phosphor sheet 11 is emitted from the position. The stimulated emission light 21 enters a light guide 22 from an incident end surface 22a of the light guide 22, and repeats total reflection in the light guide 22, and is output from an output end surface 22b of the light guide 22. The output stimulated emission light 22 is received by a photomultiplyer 23, and converted into analog image signal Sa by photoelectric conversion.

After the analog image signal Sa is logarithmically amplified by a log amplifier 26, the analog image signal Sa is sampled with a sampling interval corresponding to a spatial frequency of fs = 10 cycle/mm and digitized by an A/D converter 28, and digital image signal Sd (hereinafter, simply referred to as "image signal Sd") is output. The image signal Sd represents radiographic image information obtained by two-dimensionally scanning the storable phosphor sheet 11. As illustrated in Figure 4, the radiographic image information is obtained by moving the storable phosphor sheet 11 in a sub-scan direction (vertical direction) while the storable phosphor sheet 11 is scanned with the light beam 17 in a main scan direction (horizontal direction). The image signal Sd obtained in this manner includes information about a moire pattern 6 caused by a grid image (not illustrated) corresponding to the grid 4 in addition to information about the radiographic image 5 corresponding to the subject 3.

After the image signal Sd is temporarily stored in a storage unit 29, the image signal Sd is input to an image signal processing unit 30. The image signal processing unit 30 includes an image processing apparatus 40 for performing an image processing method in the present invention.

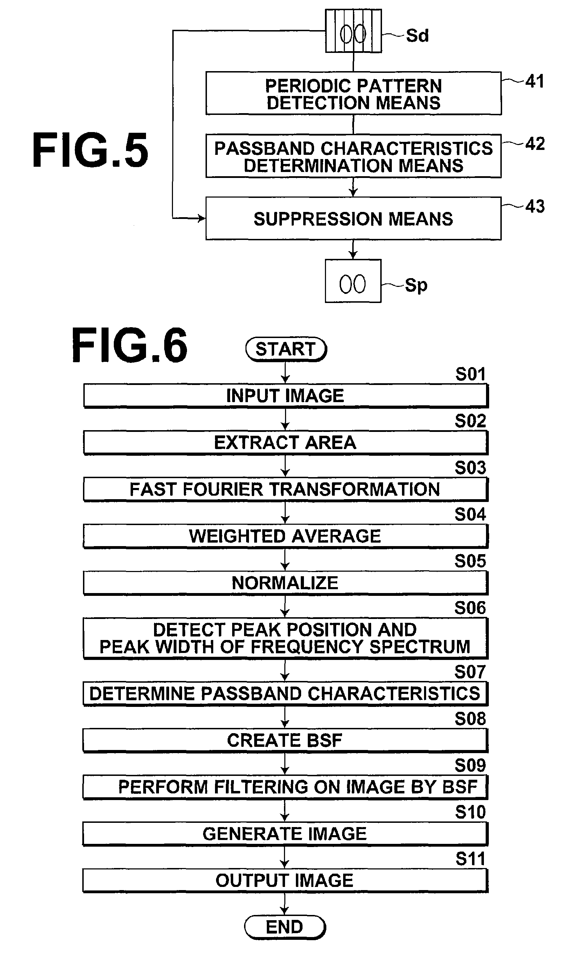

Figure 5 is a schematic diagram illustrating the configuration of the image processing apparatus 40 according to an embodiment of the present invention. The image processing apparatus 40 according to the embodiment of the present invention will be described with reference to Figure 5.

The image processing apparatus 40 according to an embodiment of the present invention includes a periodic pattern detection means 41, a passband characteristics determination means 42, and a suppression means 43. The periodic pattern detection means 41 detects a peak position and a peak width of a frequency spectrum corresponding to a periodic pattern by performing, with respect to at least one direction, frequency analysis on image signal Sd representing an image including the periodic pattern. The passband characteristics determination means 42 determines, based on the detected peak position and the detected peak width, the passband characteristics of one-dimensional filter that suppresses a spatial frequency component corresponding to the periodic pattern in the image signal. The suppression means 43 suppresses the periodic pattern in the image signal by performing one-dimensional filtering based on the determined passband characteristics.

An image processing program in an embodiment of the present invention and data to which the image processing program refers are stored in the storage unit 29 when the image processing program is installed, and loaded in a memory included in the storage unit 29 when the image processing program is started. The image processing program defines periodic pattern detection processing, passband characteristics determination processing and suppression processing, as processing performed by a central processing unit of the image signal processing unit 30, which constitutes the image processing apparatus 40. The central processing unit executes each of the aforementioned kinds of processing based on the program. Accordingly, the central processing unit of the image signal processing unit 30 functions as the periodic pattern detection means 41, the passband characteristics determination means 42, and the suppression means 43.

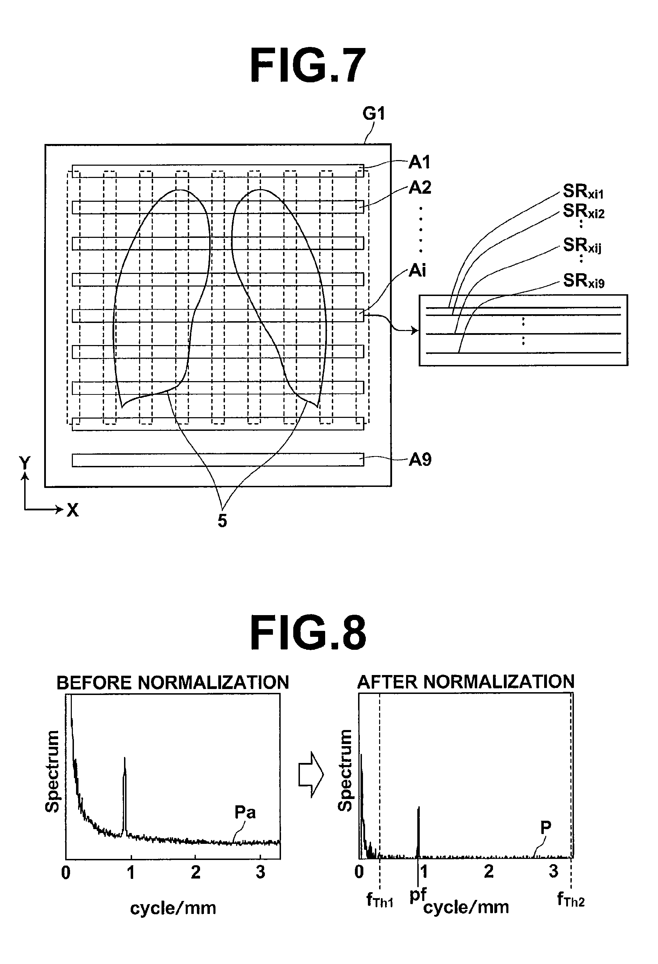

Figure 6 is a flow chart illustrating a flow of image processing in the image processing apparatus 40 according an embodiment of the present invention. Figure 7 is a diagram for explaining line-shaped sample area extraction processing by the periodic pattern detection means 41. Image processing in the image processing apparatus 40 according to the embodiment of the present invention will be described in detail with reference to Figure 6 and Figure 7.

First, as illustrated in Figure 6, image data Sd are input to the periodic pattern detection means 41 (S01). Then, the periodic pattern detection means 41 extracts n×m line-shaped areas with respect to each of x direction and y direction in an image represented by image data Sd, as illustrated in Figure 7. Since a method for extracting areas with respect to x direction and a method for extracting areas with respect to y direction are the same, only the method for extracting the areas with respect to x direction will be described here.

First, the periodic pattern detection means 41 extracts n (n = 9 in the embodiment of the present invention) rectangular areas A1 through An, which extend in x direction on image G1 represented by image data Sd (S02). Then, the periodic pattern detection means 41 obtains m (m = 9 in the embodiment of the present invention) sets of line-shaped area image data SRxij (i = 1 through n, and j = 1 through m) for each of n sets of area image data SRxi. Here, the center of area A1 in y direction is located away from the upper edge of the image by 1/30 of the height of the image. The center of area A9 in Y direction is located away from the upper edge of the image by 1/30 of the height of the image. Further, area A2 through area A7 are arranged at equal distance between area A1 and A9. The m (m = 9 in the embodiment of the present invention) sets of line-shaped area image data SRxij (i = 1 through n, and j = 1 through m) are arranged at three pixel intervals in y direction. The number of pixels in x direction is 1024.

The periodic pattern detection means 41 performs frequency analysis by performing fast Fourier transformation on each SRxij (i = 1 through n, and j = 1 through m) (S03). Specifically, fast Fourier transformation is performed on pixel string SRxij(k)(k = 0 through 1023) of each area image data SRxij. In other words, when spectrum is Pij(k)(k = 0 through 1023), and a real part and an imaginary part in an operation result obtained by fast Fourier transformation are Re(k) and Im(k), spectrum Pij (k) is calculated by the following equation:
[Expression 1]

$$Pij(k) = \sqrt{\left((Re(k))^2 + Im(k)^2\right)} \qquad \cdots (1)$$

Since the spectrum is calculated only for frequencies less than or equal to Nyquist frequency in actual processing, the number of sets of data is 1/2 of 1024, which is 512. Therefore, Pij (k') (k' = 0 through 511) will be used as a reference sign of the spectrum hereinafter. Further, the periodic pattern detection means 41 calculates spectrum Pa(k'), which is a weighted average of m×n spectra Pij(k')(i = 1 through n, and j = 1 through m) calculated for n×m sets of area image data SRxij, respectively (S04).

The left side of Figure 8 illustrates an example of spectrum Pa(k'). As the left-side diagram of Figure 8 illustrates, the obtained spectrum becomes lower as the frequency increases from a low frequency toward a high frequency, and has scattered small peaks.

Then, the periodic pattern detection means 41 normalizes the weighted-averaged spectrum Pa(k') (S05). The periodic pattern detection means 41 smoothes the spectrum Pa(k'), and calculates spectrum Pb(k'), in which small peaks in the spectrum Pa(k') are removed by smoothing. Further, the periodic pattern detection means 41 normalizes the spectrum Pa(k') by subtracting the calculated spectrum Pb(k') from the spectrum Pa (k') illustrated in the left-side diagram of Figure 8, and which was calculated from image signal Sd. Accordingly, normalized spectrum P(k') illustrated in the right-side diagram of Figure 8 is obtained. Normalization is performed to accurately evaluate by making a peak clearer.

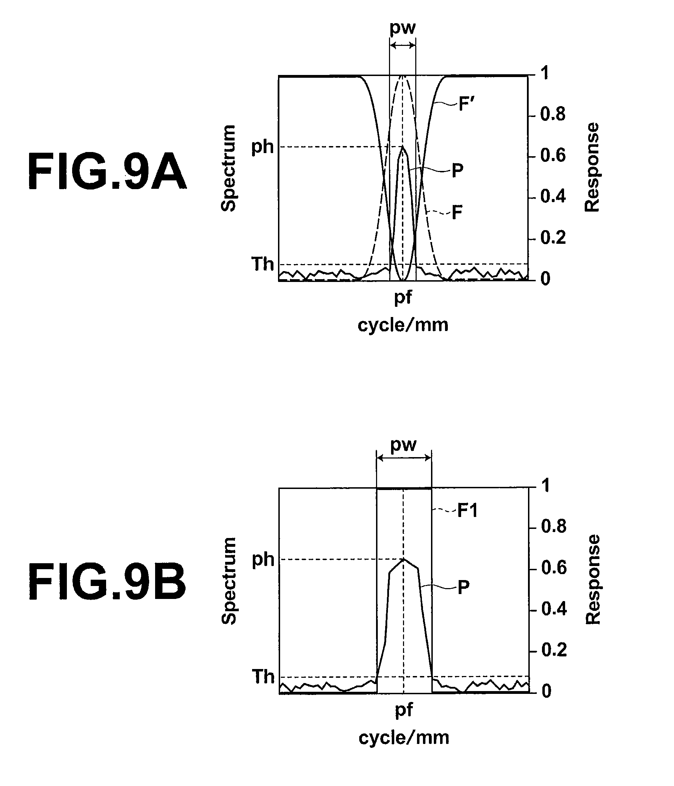

Then, the periodic pattern detection means 41 obtains a maximum frequency (peak position), which is a frequency with the maximum value of spectrum, and a peak width from the normalized spectrum P (S06). Figure 9A is a diagram for explaining processing for determining passband characteristics. In Figure 9A, normalized spectrum P on the right side of Figure 8 is enlarged. First, as the right side of Figure 8 and Figure 9A illustrate, the periodic pattern detection means 41 sets a predetermined band ($f_{Th1} \le f \le f_{Th2}$), as an evaluation band, to prevent error detection, and obtains frequency (peak position) pf, at which maximum spectrum is exhibited, and the maximum value of peak (peak height ph). Further, the periodic pattern detection means 41 obtains, as peak width pw, the width of a frequency band including the peak position, and in which spectrum is greater than or equal to predetermined threshold Th in the evaluation band. The periodic pattern detection means 41 stores the obtained peak position pf, peak width pw and peak height ph in a memory. Meanwhile, in the embodiment of the present invention, $f_{Th1}$ = 0.3 (cycle/mm) and $f_{Th2}$ = 3.3(cycle/mm), as illustrated in the right-side diagram of Figure 8. The evaluation band is set in such a manner to exclude low frequencies including many subject components and to include a frequency band less than or equal to Nyquist frequency, in which a frequency component corresponding to a moire pattern 6, which is a folding component of the grid image, is expected to be present.

Then, the passband characteristics determination means 42 determines passband characteristics, based on the obtained peak position pf and peak width pw (S07). In the embodiment of the present invention, passband characteristics (filter characteristics) are determined based on normal distribution function N of the following equation (2), which is defined by using pf, as mean $\mu$, and pw/2, as standard deviation $\sigma$. Specifically, as illustrated in Figure 9A, normal distribution function N is enlarged or reduced in such a manner that a frequency response becomes 1 at peak position pf corresponding to a periodic pattern component in spectrum P, and the function is determined as passband characteristics F. In the embodiment of the present invention, the passband characteristics determination means 42 determines passband characteristic in a similar manner also for y direction.
[Expression 2]

$$N(\mu, \sigma^2) = \frac{1}{\sqrt{2\pi}\sigma} \exp\left(-\frac{(x-\mu)^2}{2\sigma^2}\right) \qquad \cdots (2)$$

Then, the suppression means 43 creates, based on the determined passband characteristics in both x and y directions, one-dimensional band-pass filters 44 respectively by using a known method (S08). For example, methods disclosed in Takahashi and Ikehara, "Digital Filter", Baifukan (particularly, paragraph 6.3, pp. 84 through 86), Iwata and Jissen, "Introduction to Digital Filter Design", CQ Publishing Co., Ltd. (particularly, chapter 8), and the like may be applied to design of a digital filter based on filter characteristics.

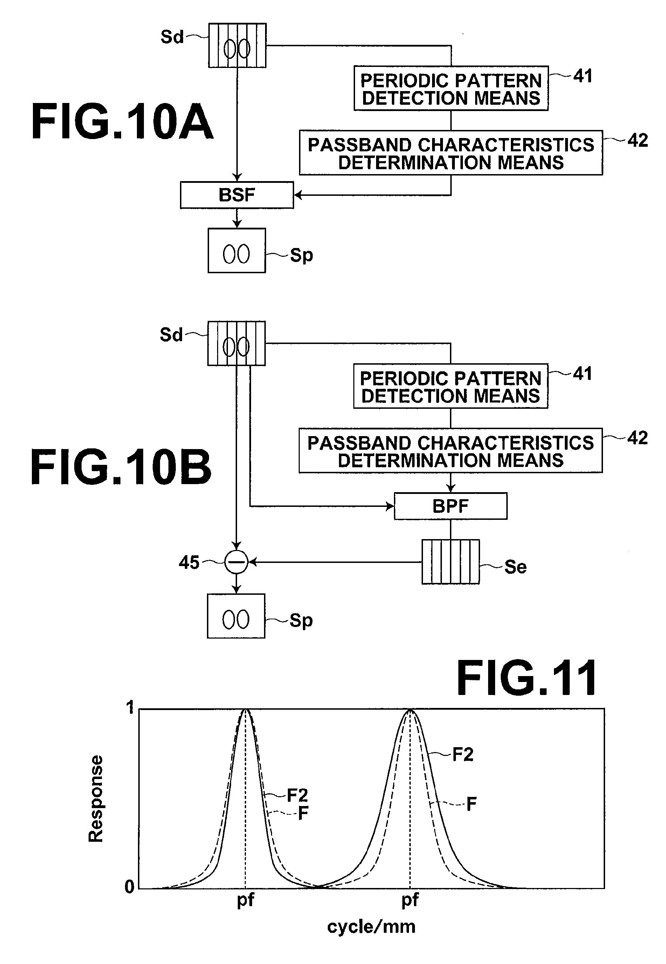

Figure 10A is a diagram for explaining processing for suppressing a periodic pattern by the suppression means 43 in the embodiment of the present invention. The suppression means 43 in the embodiment of the present invention creates, based on the passband characteristics F determined as illustrated in Figure 9A, a band stop filter (band-stop filter) BSF, as a one-dimensional band-pass filter 44. The band-stop filter BSF has passband characteristics F' in which the determined passband is removed.

Then, the suppression means 43 suppresses a periodic pattern in an image signal by performing one-dimensional filtering processing based on the determined passband characteristics (S09). Specifically, in the embodiment of the present invention, filtering processing is performed with respect to x and y directions by using one-dimensional filters (BSP) created for x and y directions, respectively.

Here, as illustrated in Figure 10A, the suppression means 43 performs filtering processing by band-stop filter BSF, which has been created based on image signal Sd, and extracts second processed signal Sp, which represents an image in which a periodic pattern has been suppressed. Further, an image corresponding to the second processed signal Sp is generated (S10), and the generated image is output (S11). After then, the output image is displayed on a display means, such as a monitor (not illustrated), or output to an output means, such as a printer (not illustrated).

According to the embodiment of the present invention, the passband characteristics of the one-dimensional filter that suppresses a spatial frequency component corresponding to a periodic pattern in an image signal are determined based on the detected peak position pf and peak width pw. Therefore, it is possible to appropriately determine, based on peak position pf and peak width pw, the passband characteristics of a one-dimensional filter that appropriately suppresses frequency components without excessively suppressing a subject component in the image. Hence, it is possible to appropriately apply image processing in the embodiment of the present invention also to a periodic pattern present in a low frequency band. Further, in the embodiment of the present invention, processing for detecting a spatial frequency component corresponding to a periodic pattern and processing for suppressing the spatial frequency component corresponding to the periodic pattern are performed one-dimensionally, and the passband characteristics of a one-dimensional filter that appropriately suppresses the frequency component are determined. Therefore, it is possible to appropriately suppress the periodic pattern based on the determined passband characteristics with a relatively low calculation load. Further, it is possible to obtain a high quality image, because only spatial frequencies corresponding to the periodic pattern are intensively suppressed.

Further, the passband characteristics determination means 42 in the embodiment of the present invention determines passband characteristics F based on a normal distribution function using peak position pf, as a mean, and a half of peak width pw, as standard deviation. Therefore, the determined passband characteristics F have the peak position pf, as the center, and smooth distribution that appropriately reflects the peak width pw. Specifically, the passband characteristics F in the embodiment of the present invention include the peak position and the passband width is wider as the peak width is wider. Therefore, it is possible to appropriately determine the passband characteristics F in such a manner to include the peak position pf of the spatial frequency component corresponding to the periodic pattern. Further, according to the embodiment of the present invention, a probability that the passband characteristics F are determined in such a manner to include the peak width pw with respect to the peak position, as the center, is high. Therefore, it is possible to appropriately determine the passband characteristics F including the spatial frequency component corresponding to the periodic pattern.

Further, the image processing apparatus 40 in the embodiment of the present invention further includes the suppression means 43, which suppresses the periodic pattern by performing one-dimensional filtering processing based on the passband characteristics that have been determined based on the image signal. Therefore, it is possible to appropriately suppress the spatial frequency component corresponding to the periodic pattern based on the determined passband characteristics F.

Next, a modified example of the embodiment of the present invention will be described.

In the aforementioned embodiment, the periodic pattern detection means 41 may set the peak position, the peak width and the peak height by using arbitrary definitions as long as representative values of the position, width and height of the peak are defined. For example, the peak width may be set at a width that is a few times (twice or three times) as wide as a band having a frequency response higher than or equal to a predetermined value.

Further, the passband characteristics determination means 42 may determine any passband characteristics as long as the passband characteristics include a peak position of a frequency component corresponding to a periodic pattern, and are determinable based on the peak position and the peak width. Figure 9B is a diagram illustrating an example in which passband characteristics are defined by a rectangular function. For example, as illustrated in Figure 9B, passband characteristics F1 may be determined by a rectangular function that passes the same frequency band as peak width pw, and in which peak position pf is the center.

Further, passband characteristics in the present invention may be determined by performing various kinds of correction on passband characteristics determined based on peak position pf and peak width pw. Next, a modified example of passband characteristics in the present invention will be described.

Generally, a low frequency band includes many subject components. Therefore, a risk of removing the subject components together with a frequency component corresponding to a periodic pattern is higher as the band-pass of the filter is wider in the low frequency band. Therefore, in a low frequency band, it is desirable that the passband characteristics include the peak of the periodic pattern and that the width of the passband is narrow. Figure 11 is a diagram illustrating a modified example of passband characteristics. As Figure 11 illustrates, the passband characteristics determination means 42 in the aforementioned embodiment may determine the passband characteristics F in such a manner that the peak position pf of the periodic pattern is included and that the width of the passband is narrower as the peak position pf is located in a lower frequency band, as a modified example of the embodiment of the present invention. For example, peak position pf may be stored in the storage unit 29 in such a manner to be linked with a passband width in passband characteristics F. When the periodic pattern detection means 41 detects peak position pf, the passband characteristics F in the aforementioned embodiment may be corrected by enlarging or reducing in the width direction so that the width becomes a passband width linked with the detected peak position pf. Further, a function obtained as a result may be determined as passband characteristics F2. In this case, it is possible to appropriately remove only a spatial frequency component corresponding the periodic pattern, such as moire, while many subj ect components included in a low frequency component are maintained, compared with conventional techniques.

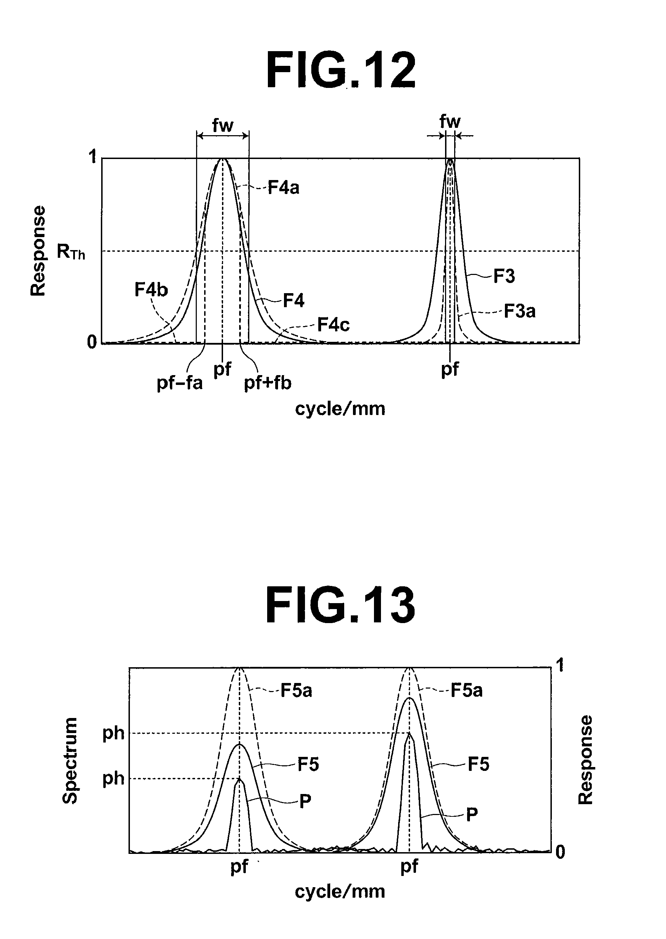

Further, it is not desirable to perform filtering processing by a band-pass (band-stop) filter that passes (or stops) only an extremely sharp band, because that causes generation of an artifact or the like in an image. Therefore, the passband characteristics determination means 42 may set a lower limit in the width of the passband. Figure 12 is a diagram illustrating a modified example of passband characteristics in the aforementioned embodiment. When the width of the passband characteristics F is less than a predetermined value, for example, when a periodic pattern is a too sharp peak or the like, a function in which the passband characteristics F in the aforementioned embodiment are corrected so that the width of the passband becomes wider may be determined as passband characteristics. For example, as illustrated in Figure 12, when passband width fw in predetermined frequency response $R_{Th}$ in the passband characteristics F3a that have been determined based on peak position pf and peak width pw is less than a predetermined value, passband characteristics in which the width of the passband characteristics F is corrected to become wider by a predetermined ratio may be determined as passband characteristics F3. Alternatively, the peak width pw may be corrected to become wider at a predetermined ratio, and passband characteristics may be determined based on the corrected peak width.

In that case, it is possible to prevent generation of an artifact caused by filtering processing based on passband characteristics in which a passband is narrow. Therefore, it is possible to generate a higher quality image.

Further, it is not desirable to perform filtering processing by a band-pass (band-stop) filter that passes a too wide band, because there is a risk of removing information, such as subject components, which is important in diagnosis based on an image, together with a component corresponding to a periodic pattern. Therefore, the passband characteristics determination means 42 may set an upper limit in the width of the passband.

For example, when the width fw of the passband characteristics F is greater than a predetermined value, the passband characteristics F may be corrected in such a manner that the width of the passband characteristics becomes narrower, and the corrected passband characteristics may be determined as the passband characteristics. For example, as Figure 12 illustrates as passband characteristics F4, when width fw of the passband at predetermined frequency response $R_{th}$ of passband characteristics F4a that have been determined based on peak position pf and peak width pw is larger than a predetermined value, the width of passband characteristics F4a may be corrected so that the width becomes narrower at a predetermined ratio, and the corrected passband characteristics may be used as the passband characteristics F4. Alternatively, when peak width pw is wider than a predetermined value, the peak width pwmay be reduced at a predetermined ratio, and passband characteristics may be determined based on the corrected peak width pw. Further, the passband characteristics F4a calculated by using the peak width pw may be corrected so that frequency response in a lower frequency band than a frequency that is lower than peak position pf by predetermined value fa becomes 0, and the corrected passband characteristics may be determined as passband characteristics F4b. Specifically, as illustrated in Figure 12, passband characteristics F4b may define the frequency response in a frequency band lower than frequency (pf-fa), as 0, and frequency response higher than or equal to frequency (pf-fa) by F4a. Similarly, correction for narrowing the passband may be performed not only on the low frequency side but also on the high frequency side. For example, passband characteristics F4a or F4b may be corrected to passband characteristics F4c in which frequency response in a higher frequency band than frequency (pf+fb) that is higher than a peak position of the passband characteristics F4a or F4b by predetermined value fb is 0, and frequency response less than or equal to frequency (pf+fb) is defined by passband characteristics F4a.

In that case, it is possible to reduce the risk of removing information, such as subject components, which is important in diagnosis based on an image, together with a component corresponding to a periodic pattern, and such risk being caused by performance of filtering processing based on passband characteristics having a wide passband. Therefore, it is possible to generate a higher quality image.

Further, the passband characteristics determination means 42 may determine passband characteristics also based on the peak height (magnitude of spectrum). Figure 13 is a diagram illustrating a modified example of the embodiment of the present invention. Figure 13 is a diagram for explaining an example in which passband characteristics F5 are determined by correcting passband characteristics F5a that have been determined based on peak position pf and peak width pw, and the passband characteristics F5a are corrected in such a manner that the frequency response of the passband characteristics is lower as the peak height ph (magnitude of spectrum) is lower. As passband characteristics F5 in Figure 13 illustrate, when height ph of the spectrum is small, even if not an entire frequency band including a frequency component corresponding to a periodic pattern is suppressed (a part of the frequency component corresponding to the periodic pattern remains in the image signal), it is possible to suppress the periodic pattern in such a manner that the quality of the image is sufficient as an image for diagnosis. In this case, passband characteristics may be determined by using an arbitrary method as long as the band-pass frequency response by the band-pass filter is higher as peak height ph is higher and the band-pass frequency response by the band-pass filter is lower as peak height ph is lower.

Further, the passband characteristics determination means 42 may determine different passband characteristics based on the imaging condition of an image. For example, passband characteristics appropriate for an imaged region and an imaging condition may be determined in advance, and the passband characteristics may be stored in the storage unit 29 in such a manner to be linked with each imaged region and each imaging condition. Then, the passband characteristics determination means 42 may identify the passband characteristics linked with the imaged region and the imaging condition of an image, which is a target of processing. Further, the passband characteristics determination means 42 may perform processing for suppressing a periodic pattern by using the identified passband characteristics.

In that case, it is possible to appropriately perform suppression processing based on passband characteristics appropriate for the imaged region and the imaging condition. Therefore, it is possible to extract a second processed signal, in which a frequency component corresponding to the periodic pattern is appropriately suppressed. Consequently, it is possible to generate a higher quality image in which the frequency component corresponding the periodic pattern is suppressed.

Here, the imaged region and the imaging condition of the image may be obtained by using an imaging menu, such as the imaging condition and the imaged region, which is specified at an imaging apparatus of the original image. Alternatively, imaged region information, such as an organ of the patient and a lesion, which has been input by a user through a manual operation may be used. Further, the image processing apparatus 40 may include a region extraction means, which is not illustrated. The region extraction means extracts an imaged region of an image, and extracted imaged region information may be used. As a method for extracting imaged region information, any method may be adopted as long as a region, a lesion or the like can be extracted. For example, methods disclosed in Japanese Unexamined Patent Publication No. 2002-109548 and Japanese Unexamined Patent Publication No. 2003-006661, which are proposed by the applicant of the present application, may be used. In the methods, a thorax is automatically detected by performing template matching using a template that is substantially similar to the outline of an average cardiothorax, as reference.

In such a case, the passband characteristics determination means 42 does not need a user's input operation of an imaged region, and can extract an imaged region based on the result of automatic extraction. Therefore, it is possible to appropriately determine passband characteristics based on the extracted imaged region. Hence, a user can easily generate a high quality image in which a frequency component corresponding to a periodic pattern is suppressed based on the imaged region. Further, when the passband characteristics determination means 42 automatically extracts an imaged region or an imaging condition from an imaging menu, and determines passband characteristics based on the result of automatic extraction, the same effect is achievable.

Further, when imaging conditions are substantially the same, as in the case of consecutively suppressing a periodic pattern in the same region at the same apparatus or the like, a band-pass filter (band-stop filter) may be determined by performing, on image signal Sd of the first subject, image processing according to an embodiment of the present invention. Further, the same filter may be applied to the following image signals. When imaging conditions are substantially the same, it is considered that images including a similar periodic pattern are obtained. Therefore, it is possible to sufficiently suppress the periodic pattern by using a band-pass filter (band-stop filter) determined for a representative image. Further, since it is possible to save time and work for determining passband characteristics for each image, high efficiency is achievable.

The suppression means 43 may perform suppression processing by using any combination of a band-pass filter and a band-stop filter in a horizontal direction and in a vertical direction and in any order of filtering processing as long as an image signal (Sp in the embodiment of the present invention) in which a periodic pattern has been suppressed based on determined passband characteristics is finally obtainable for the image signal Sd. For example, a band-pass filter (band pass filter) BPF having passband characteristics F, which pass determined passband, may be created based on the determined passband characteristics F. Figure 10B is a diagram illustrating a modified example of suppression processing in the aforementioned embodiment. As illustrated in Figure 10B, image signal Se (a periodic pattern image extracted from image signal Sd) is generated by performing filtering processing on image signal Sd by using band-pass filter BPF. Further, the image signal Se is subtracted from the image signal Sd. Accordingly, it is possible to suppress the periodic pattern in a similar manner to the aforementioned embodiment. In this case, the suppression means 43 includes band-pass filter BPF and a subtractor 45. The suppression means 43 performs filtering processing on image signal Sd by using the band-pass filter BPF, and calculates periodic pattern image signal Se, which is an extracted periodic pattern component. After then, the subtractor 45 extracts processed signal Sp, which represents an image in which the periodic pattern is suppressed, by subtracting the periodic pattern image signal Se.

In the aforementioned embodiment, the periodic pattern detection means 41 may obtain image signals on plural lines only for a representative area instead of plural sample areas. Further, the periodic pattern detection means 41 may obtain a weighted average by performing frequency analysis, and obtain spectrum Pa. Alternatively, the periodic pattern detection means 41 may obtain an image signal on a line, and obtain a spectrum by performing frequency analysis only for this image signal. The obtained spectrum may be used instead of normalized spectrum Pa.

In the aforementioned embodiment, the periodic pattern detection means 41 may omit normalization processing. However, it is desirable to perform normalization processing, because it is possible to accurately detect information, such as a peak width, a peak value and a peak height.

In the embodiments of the present invention, a case in which the present invention is applied to a radiographic image readout apparatus (CR: Computed Radiography) has been described. In the radiographic image readout apparatus, excitation light, such as a laser beam, is output to a storable phosphor sheet, and stimulated emission light is generated. Photoelectric conversion is performed on the stimulated emission light to obtain an image signal. The present invention is not limited to the aforementioned embodiments, and may be applied to any type of radiographic image readout apparatus using a grid. For example, the present invention may be applied to a radiographic image readout apparatus using a radiation solid-state detector (hereinafter referred to as a radiation solid-state detector of "light conversion type and indirect conversion type"), and in which plural photoelectric conversion elements, each corresponding to a pixel, are two-dimensionally formed on an insulation substrate. A phosphor layer (scintillator) that converts radiation carrying image information into visible light by irradiation with the radiation is deposited and constitutes the radiation solid-state detector formed on the radiation image readout apparatus. Further, the present invention may be applied to a radiography apparatus using a radiation solid-state detector (hereinafter referred to as a radiation solid-state detector of "direct conversion type"). Plural charge collecting electrodes, each corresponding to a pixel, are two-dimensionally formed on an insulation substrate in a radiographic image readout apparatus. A radiation conductor that generates charges carrying image information by irradiation with radiation carrying the image information is deposited, and constitutes the radiation solid-state detector formed on the two-dimensional image readout apparatus.

Further, the present invention may be applied to a radiographic image readout apparatus using various kinds of radiation solid-state detector. As a radiation solid-state detector of light conversion type, radiation solid-state detectors disclosed, for example, in Japanese Unexamined Patent Publication No. 59(1984)-211263, Japanese Unexamined Patent publication No. 2 (1990)-164067, PCT International Publication No. 92/006501, Signal, noise, and read out considerations in the development of amorphous silicon photodiode arrays for radiotherapy and diagnostic x-ray imaging, L.E.Antonuk et.al, University of Michigan, R.A. Street Xerox, PARC, SPIE Vol.1443 Medical Imaging V; Image Physics(1991), pp.108-119, and the like may be adopted.

Meanwhile, as a radiation solid-state detector of direct conversion type, radiation solid-state detectors disclosed for example in (i) a radiation solid-state detector, the thickness of which in the transmission direction of radiation is set about ten times as thick as an ordinary one (MATERIAL PARAMETERS IN THICK HYDROGENATED AMORPHOUS SILICON RADIATION DETECTORS, Lawrence Berkeley Laboratory.University of California, Berkeley. CA 94720 XeroxParc.Palo Alto.CA 94304), or (ii) a radiation solid-state detector, in which two or more layers are deposited in the transmission direction of radiation with a metal plate therebetween (Metal/Amorphous Silicon Multilayer Radiation Detectors, IEEE TRANSACTIONS ON NUCLEAR SCIENCE, VOL. 36, NO.2, APRIL 1989), or (iii) a radiation solid-state detector using CdTe or the like (Japanese Unexamined Patent Publication No. 1(1989)-216290) or the like may be adopted.

Each of the aforementioned embodiments is only an example, and all of the descriptions should not be used to interpret the technical scope of the present invention in a limited manner. Further, the system configuration, the hardware configuration, the process flow, the module configuration, the specific content of processing and the like may be modified in various manners without departing from the gist of the present invention. Such modifications remain in the technical scope of the present invention.

**Claims**

1. An image processing apparatus comprising:

    a periodic pattern detection means that detects a peak position and a peak width of a frequency spectrum corresponding to a periodic pattern by performing, with respect to at least one direction, frequency analysis on an image signal representing an image including the periodic pattern; and

    a passband characteristics determination means that determines, based on the detected peak position and the detected peak width, the passband characteristics of a one-dimensional filter suppressing a spatial frequency component corresponding to the periodic pattern in the image signal.

2. The image processing apparatus, as defined in Claim 1, wherein the passband characteristics determination means determines the passband characteristics in such a manner that the peak position is included and that a passband width is wider as the peak width is wider.

3. The image processing apparatus, as defined in Claim 2, wherein the passband characteristics determination means determines the passband characteristics by a normal distribution function defined by the peak position and the peak width.

4. The image processing apparatus, as defined in any one of Claims 1 to 3, wherein the passband characteristics determination means determines the passband characteristics in such a manner that a passband width is narrower as the peak position is located in a lower frequency band.

5. The image processing apparatus, as defined in any one of Claims 1 to 4, wherein the passband characteristics determination means determines the passband characteristics based on the height of the peak.

6. The image processing apparatus, as defined in any one of Claims 1 to 5, the apparatus further comprising:

   a suppression means that suppresses the periodic pattern in the image signal by performing one-dimensional filtering based on the determined passband characteristics.

7. The image processing apparatus, as defined in any one of Claims 1 to 6, wherein the passband characteristics determination means sets an upper limit or a lower limit of a passband width in the passband characteristics.

8. The image processing apparatus, as defined in any one of Claims 1 to 7, wherein the passband characteristics determination means determines the passband characteristics in such a manner that the passband characteristics differ depending on an imaging condition of the image.

9. The image processing apparatus, as defined in any one of Claims 1 to 8, wherein the passband characteristics determination means determines the passband characteristics by a rectangular function having a width wider than or equal to the peak width, and the center of the rectangular function being the peak position.

10. An image processing method comprising the steps of:

    detecting a peak position and a peak width of a frequency spectrum corresponding to a periodic pattern by performing, with respect to at least one direction, frequency analysis on an image signal representing an image including the periodic pattern; and
    determining, based on the detected peak position and the detected peak width, the passband characteristics of a one-dimensional filter suppressing a spatial frequency component corresponding to the periodic pattern in the image signal.

11. An image processing program for causing a computer to function as:

    a periodic pattern detection means that detects a peak position and a peak width of a frequency spectrum corresponding to a periodic pattern by performing, with respect to at least one direction, frequency analysis on an image signal representing an image including the periodic pattern; and
    a passband characteristics determination means that determines, based on the detected peak position and the detected peak width, the passband characteristics of a one-dimensional filter suppressing a spatial frequency component corresponding to the periodic pattern in the image signal.

# FIG.1

# FIG.2

# FIG.3

Block diagram (right side):

- Sa → log — 26
- A/D — 28
- Sd
- STORAGE UNIT — 29
- Sd
- IMAGE SIGNAL PROCESSING UNIT — 30
  - IMAGE PROCESSING APPARATUS — 40

Labels: 16, 17, 19, 20, 23, 22, 18, 22b, 24, 21, 10, X, Y, 22a, 11, 15

MAIN SCAN DIRECTION
(HORIZONTAL DIRECTION)

SUB-SCAN DIRECTION
(VERTICAL DIRECTION)

11

# FIG.4

**FIG.5**

~Sd

| PERIODIC PATTERN DETECTION MEANS | ~41 |

| PASSBAND CHARACTERISTICS DETERMINATION MEANS | ~42 |

| SUPPRESSION MEANS | ~43 |

00 ~Sp

**FIG.6**

START

INPUT IMAGE — S01

EXTRACT AREA — S02

FAST FOURIER TRANSFORMATION — S03

WEIGHTED AVERAGE — S04

NORMALIZE — S05

DETECT PEAK POSITION AND PEAK WIDTH OF FREQUENCY SPECTRUM — S06

DETERMINE PASSBAND CHARACTERISTICS — S07

CREATE BSF — S08

PERFORM FILTERING ON IMAGE BY BSF — S09

GENERATE IMAGE — S10

OUTPUT IMAGE — S11

END

# FIG.7

# FIG.8

# FIG.9A

# FIG.9B

**FIG.10A**

Sd

PERIODIC PATTERN
DETECTION MEANS — 41

PASSBAND CHARACTERISTICS
DETERMINATION MEANS — 42

BSF

00 — Sp

**FIG.10B**

Sd

PERIODIC PATTERN
DETECTION MEANS — 41

PASSBAND CHARACTERISTICS
DETERMINATION MEANS — 42

BPF

45 — (−)

Se

00 — Sp

**FIG.11**

Response

F2
F

F2
F

pf                pf

cycle/mm

# FIG.12

# FIG.13

<table>
<tr><td colspan="2" align="center">**INTERNATIONAL SEARCH REPORT**</td><td colspan="2">International application No.<br>PCT/JP2012/002012</td></tr>
</table>

**A. CLASSIFICATION OF SUBJECT MATTER**
*A61B6/00*(2006.01)i, *G06T5/10*(2006.01)i

According to International Patent Classification (IPC) or to both national classification and IPC

**B. FIELDS SEARCHED**

Minimum documentation searched (classification system followed by classification symbols)
A61B6/00-6/14, G06T5/10

Documentation searched other than minimum documentation to the extent that such documents are included in the fields searched
Jitsuyo Shinan Koho        1922-1996    Jitsuyo Shinan Toroku Koho    1996-2012
Kokai Jitsuyo Shinan Koho    1971-2012    Toroku Jitsuyo Shinan Koho    1994-2012

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2009-195512 A  (Fujifilm Corp.),<br>03 September 2009 (03.09.2009),<br>entire text; fig. 1 to 8<br>(Family: none) | 1-11 |
| Y | JP 10-246776 A  (Mitsubishi Electric Corp.),<br>14 September 1998 (14.09.1998),<br>entire text; fig. 1 to 14<br>(Family: none) | 1-11 |
| A | US 6269176 B1  (Eastman Kodak Co.),<br>31 July 2001 (31.07.2001),<br>entire text; fig. 1 to 16<br>(Family: none) | 1-11 |

☐ Further documents are listed in the continuation of Box C.          ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>25 June, 2012 (25.06.12) | Date of mailing of the international search report<br>03 July, 2012 (03.07.12) |
|---|---|
| Name and mailing address of the ISA/<br>Japanese Patent Office | Authorized officer |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2003150954 A
- US 6269176 B
- JP 2009195512 A
- JP 2002109548 A
- JP 2003006661 A
- JP 59211263 A
- JP 2164067 A
- JP 4006501 W
- JP 1216290 A

### Non-patent literature cited in the description

- **L.E.ANTONUK.** SPIE Vol.1443 Medical Imaging V. *Image Physics,* 1991, vol. 1443, 108-119
- Metal/Amorphous Silicon Multilayer Radiation Detectors. *IEEE TRANSACTIONS ON NUCLEAR SCIENCE,* April 1989, vol. 36 (2